# EUROPEAN PATENT APPLICATION

(11) **EP 4 201 359 A1**
(43) Date of publication of application: **28.06.2023**
(21) Application number: 21861985.6
(22) Date of filing: 23.08.2021
(51) Int. Cl.: A61B 18/22, A61B 18/24, A61M 1/00, A61B 18/00, A61B 18/20

(54) **FAT-REMOVING SURGICAL INSTRUMENT**

(30) Priority: 24.08.2020 KR 20200106294
(71) Applicant: Rhim, Si Youn, Seoul 04747 (KR)
(72) Inventor: Rhim, Si Youn, Seoul 04747 (KR)
(74) Representative: Botti, Mario
(86) International application number: PCT/KR2021/011165
(87) International publication number: WO 2022/045688

(57) **Abstract**

The present disclosure relates to a fat-removing surgical instrument. The present disclosure additionally arranges/provides, on a lipolyzed fat suction passage of a wave-guide tube, an optical fiber support which is made from an insulating material and which supports optical fibers in a restrained state while fixed to the wave-guide tube, so that the optical fibers are prevented from being deformed and coming in contact with the wave-guide tube, and thus, the optical fibers are induced, even though a variety of factors (for example, weight, contact with human tissue and the like) are applied to the optical fibers during fat removal surgery, to effectively avoid coming in direct contact with the wave-guide tube and being damaged thereby, while a series of deformations (for example, a curved shape and the like) are inhibited/controlled, on the basis of forceful restraint of the optical fiber support and, as a result, subjects (for example, medical teams for fat removal surgery and the like) who carry out fat removal surgery can be supported so as to be capable of normally carrying out effective fat removal surgery in which high frequency/laser simultaneous oscillation, lipolyzed fat simultaneous suction and the like can be performed together, while easily avoiding serious problems due to the excessive inflow/diffusion of broken optical fiber scraps into a human body.

## Description

### [Technical Field]

The present disclosure relates to a fat-removing surgical instrument, and more particularly, to a fat-removing surgical instrument in which 'an optical fiber support made of an insulating material and fixed to a wave-guide tube to support an optical fiber in a restrained state to prevent deformation of the optical fiber and contact with the wave-guide tube' is additionally placed/provided on a lipolyzed fat suction passage of the wave-guide tube to induce the optical fiber to effectively avoid the direct contact with the wave-guide tube and its consequential damage by suppressing/regulating a series of deformations (for example, a curved shape) based on the forceful restraint of the optical fiber support even though a variety of factors (for example, weight, contact with tissue in human body) are applied to the optical fiber during the fat removal surgery, thereby supporting a fat removal surgical operator (for example, a fat removal surgery medical professional) to normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

### [Background Art]

Recently, with the growing social interest in health and beauty, various types of fat-removing surgical instruments have been developed/widespread.

For example, Korean Patent Publication No. 10-2014-133786 (titled handpiece for lipolysis) (published November 20, 2014), Korean Patent Publication No. 10-2015-43511 (titled kit of parts and apparatus for liposuction and lipofilling including the same) (published April 22, 2015), and Korean Patent Publication No. 10-2017-80184 (titled medical laser handpiece with high frequency heating function) (published July 10, 2017) disclose examples of fat-removing surgical instruments according to related art in more detail.

Meanwhile, as shown in FIG. 1, under the regime of Korean Patent No. 10-1247376 (titled handpiece for lipolysis·liposuction surgery) (published March 26, 2013) (hereinafter referred to as '1247376 patent'), the handpiece 4 assumes a combined configuration of a wave-guide tube 2 coupled to a handle 1 and extended in the forward direction, and an optical fiber 3 inserted at the center of the wave-guide tube 2 and having a rear end extended in the rearward direction of the handle 2.

Under this situation, when a surgery for fat removal (for example, lipolysis, liposuction) in a subject's body B is performed, high frequency is emitted from the wave-guide tube 2 and a laser is emitted from the optical fiber 3, and eventually, unnecessary fat in the subject's body B may be removed under the influence of the high frequency and laser.

Meanwhile, as shown in FIG. 2, under the regime of the applicant's Korean Patent No. 10-2058016 (titled fat-removing surgical instrument) (published December 20, 2019) (hereinafter referred to as '2058016 patent'), the fat-removing surgical instrument 10 assumes a systematically combined configuration of a handle 11, a wave-guide tube 12 extended and installed in the frontward direction of the handle 11, defining a lipolyzed fat suction passage 14 for the suction of lipolyzed fat and configured to emit high frequency during the fat removal surgery, and an optical fiber 13 installed on the lipolyzed fat suction passage 14 of the wave-guide tube 12, and having an end exposed in the frontward direction of the wave-guide tube 12 to emit a laser during the fat removal surgery. In this case, a high frequency cable (not shown) may be additionally embedded in the wave-guide tube 12 and electrically connected to a fat removal control terminal 30 to emit the high frequency.

Under this situation, when the fat removal surgery in the subject's body B starts, the fat removal control terminal 30 operates a high frequency processing module, a laser processing module and a lipolyzed fat suction processing module, for example, to simultaneously perform the procedure of applying the high frequency to the wave-guide tube 12, the procedure of applying the laser to the optical fiber 13 and the procedure of sucking up lipolyzed fat through the lipolyzed fat suction passage 14, thereby allowing the fat removal surgical operator (for example, a fat removal surgery medical professional) to perform <effective fat removal surgery involving heating a localized area using the laser to rupture, breaking down the ruptured area using the high frequency and sucking up the broken fat crystals quickly>, for example, under an environment in which simultaneous emission of high frequency and laser and simultaneous suction of lipolyzed fat are performed together.

In this instance, the '2058016 patent' further includes an insulation tube 100 compared to the '1247376 patent'.

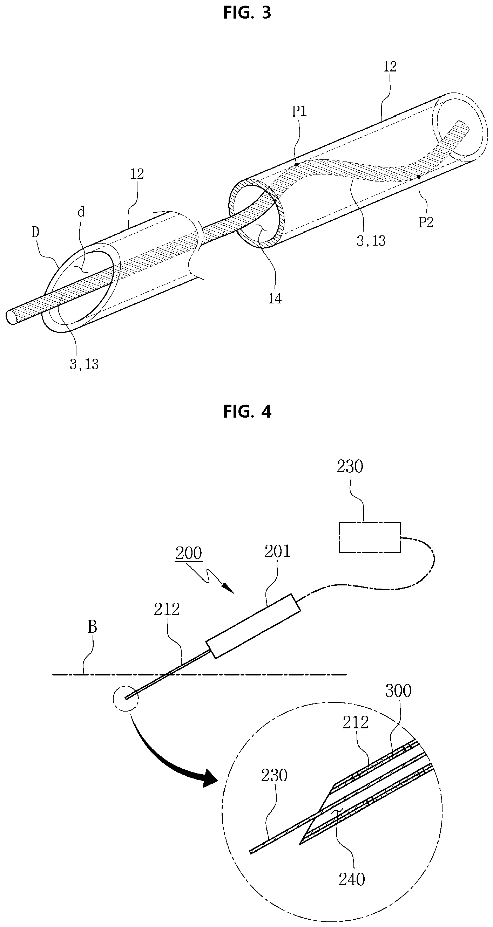

Meanwhile, under the existing regime, when the fat removal surgery is performed, as shown in FIG. 3, the optical fibers 3, 13 extended along the wave-guide tubes 2, 12 unavoidably suffer a series of deformations (for example, a curved shape) due to a variety of factors (for example, weight, contact with tissue in human body).

In this instance, when the optical fibers 3, 13 unavoidably suffer a series of deformations (for example, a curved shape), the corresponding optical fibers 3, 13 encounter a very serious situation such as direct contact with the wave-guide tubes 2, 12 (or an insulation tube in the wave-guide tube) at different points (P1, P2, ...).

When the optical fibers 3, 13 come into direct contact with the wave-guide tubes 2, 12 (or the insulation tube in the wave-guide tube), strong high frequency emitted from the wave-guide tubes 2, 12 is directly applied to the corresponding optical fibers 3, 13, causing a serious problem that the optical fibers 3, 13 break down into pieces and are destroyed due to electromagnetic shocks of the direct high frequency.

Obviously, when the optical fibers 3, 13 break down into pieces and are destroyed by the strong electromagnetic shocks applied from the high frequency from the wave-guide tubes 2, 12, the broken optical fiber scrap shows reckless infiltration/propagation behaviors into the subject's body, and eventually, the recklessly infiltrated/propagated optical fiber scrap causes a very serious problem with severe damage to the tissues in the body B.

Meanwhile, as shown in FIG. 3, under the existing regime, as described above, the optical fibers 3, 13 go over a transverse area d of the wave-guide tubes 2, 12 and their ends are exposed in the forward direction of the wave-guide tube 12.

Under a situation in which the ends of the optical fibers 3, 13 go over the transverse area d of the wave-guide tubes 2, 12 inside the human body and are exposed in the frontward direction, a large volume of strong high frequency is outputted in the frontward direction of the wave-guide tubes 2, 12 along the arrow from the tips D of the wave-guide tubes 2, 12 inside the human body.

Under a situation in which a large volume of strong high frequency is outputted from the tips D of the wave-guide tubes 2, 12, strong electromagnetic shocks of the corresponding high frequency are applied to the optical fibers 3, 13, causing a serious problem that the optical fibers 3, 13 break down into pieces and are destroyed.

Obviously, also in this case, the broken optical fiber scrap shows reckless infiltration/propagation behaviors into the subject's body, and eventually, the recklessly infiltrated/propagated optical fiber scrap causes a very serious problem with severe damage to the tissues in the body B.

In summary, the '2058016 patent' and '1247376 patent' correspond to imperfect technology that causes damage to the tissues in the body B due to the optical fiber scrap broken by high frequency, and unless any epoch-making action is taken, the fat removal surgical operator (for example, a fat removal surgery medical professional) cannot practically apply the '2058016 patent' and '1247376 patent' to <fat removal surgery in the body B>.

### [RELATED LITERATURES]

### [Patent Literature]

(Patent Literature 1) Korean Patent No. 1 0-1247376 (titled handpiece for lipolysis·liposuction surgery) (published March 26, 2013)
(Patent Literature 2) Korean Patent No. 1 0-2058016 (titled fat-removing surgical instrument) (published 2019.12.20)
(Patent Literature 3) Korean Patent Publication No. 10-2014-133786 (titled handpiece for lipolysis) (published November 20, 2014)
(Patent Literature 4) Korean Patent Publication No. 10-2015-43511 (titled kit of parts and apparatus for liposuction and lipofilling including the same) (published April 22, 2015)
(Patent Literature 5) Korean Patent Publication No. 10-2017-80184 (titled medical laser handpiece with high frequency heating function) (published July 10, 2017)

### [Disclosure]

### [Technical Problem]

Therefore, the present disclosure is directed to additionally placing/providing 'an optical fiber support made of an insulating material and fixed to a wave-guide tube to support an optical fiber in a restrained state to prevent deformation of the optical fiber and contact with the wave-guide tube' on a lipolyzed fat suction passage of the wave-guide tube to induce the optical fiber to effectively avoid the direct contact with the wave-guide tube and its consequential damage by suppressing/regulating a series of deformations (for example, a curved shape) based on the forceful restraint of the optical fiber support even though a variety of factors (for example, weight, contact with tissue in human body) are applied to the optical fiber during the fat removal surgery, thereby supporting a fat removal surgical operator (for example, a fat removal surgery medical professional) to normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

These and other objectives of the present disclosure will be apparent from the following detailed description and the accompanying drawings.

### [Technical Solution]

To achieve the above-described objective, the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes an optical fiber hold pack extended along the wave-guide tube and covering around the optical fiber to restrain deformation of the optical fiber; and a hold pack fixing block protruded from the optical fiber hold pack and fixed to the wave-guide tube to induce the optical fiber received in the optical fiber hold pack to securely hang at a center of the lipolyzed fat suction passage.

Additionally, another aspect of the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes an optical fiber hold piece covering around the optical fiber while occupying part of the optical fiber to restrain deformation of the optical fiber; and a hold piece fixing block protruded from the optical fiber hold piece and fixed to the wave-guide tube to induce the optical fiber received in the optical fiber hold piece to securely hang at a center of the lipolyzed fat suction passage.

Additionally, another aspect of the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes a body block fixed to the wave-guide tube and protruded from the wave-guide tube toward the lipolyzed fat suction passage; and an optical fiber passing guide hole formed by making part of the body block open, through which the optical fiber passes through the body block, to induce the optical fiber to securely hang at a center of the lipolyzed fat suction passage with deformation being restrained by the body block.

Additionally, another aspect of the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes an optical fiber hold ring having a ring shape, part of which is open, and covering around the optical fiber to restrain deformation of the optical fiber; and a base block integrally connected with the optical fiber hold ring and fixed to the wave-guide tube to induce the optical fiber passing through the optical fiber hold ring to securely hang at a center of the lipolyzed fat suction passage.

Additionally, another aspect of the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes an optical fiber hold pack extended along the wave-guide tube and covering around the optical fiber to restrain deformation of the optical fiber; and a hold pack fixing string connected to the optical fiber hold pack and fixed to the wave-guide tube to induce the optical fiber received in the optical fiber hold pack to securely hang at a center of the lipolyzed fat suction passage.

Additionally, another aspect of the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes an optical fiber hold piece covering around the optical fiber while occupying part of the optical fiber to restrain deformation of the optical fiber; and a hold piece fixing string connected to the optical fiber hold piece and fixed to the wave-guide tube to induce the optical fiber received in the optical fiber hold piece to securely hang at a center of the lipolyzed fat suction passage.

Additionally, another aspect of the present disclosure discloses a fat-removing surgical instrument including a wave-guide tube defining a lipolyzed fat suction passage for suction of lipolyzed fat and configured to emit high frequency during a fat removal surgery; an optical fiber installed on the lipolyzed fat suction passage of the wave-guide tube and configured to emit a laser during the fat removal surgery; and an optical fiber support made of an insulating material and fixed to the wave-guide tube to support the optical fiber in a restrained state to prevent deformation of the optical fiber and contact with the wave-guide tube, wherein the optical fiber support includes a plurality of optical fiber fixing strings continuously wound around the optical fiber to a form a hank to restrain deformation of the optical fiber, and having two ends fixed to the wave-guide tube to induce the optical fiber to securely hang at a center of the lipolyzed fat suction passage.

Further, another aspect of the present disclosure discloses a fat-removing surgical instrument including a wave-guide tube defining a lipolyzed fat suction passage for suction of lipolyzed fat and configured to emit high frequency during a fat removal surgery; and an optical fiber installed on the lipolyzed fat suction passage of the wave-guide tube and configured to emit a laser during the fat removal surgery, wherein an inner insulation tube of an insulating material is installed at a center of the lipolyzed fat suction passage and covers around the optical fiber to restrain deformation of the optical fiber, a fixing frame of an insulating material is installed on an outer side of the inner insulation tube and protrudes from the inner insulation tube to fix the corresponding inner insulation tube on the lipolyzed fat suction passage, and the optical fiber is inserted into the inner insulation tube and securely hangs at the center of the lipolyzed fat suction passage.

### [Advantageous Effects]

Since the present disclosure additionally places/provides 'the optical fiber support made of an insulating material and fixed to the wave-guide tube to support the optical fiber in a restrained state to prevent deformation of the optical fiber and contact with the wave-guide tube' on the lipolyzed fat suction passage of the wave-guide tube, under the embodiment environment of the present disclosure, it is possible to effectively avoid the direct contact of the optical fiber with the wave-guide tube and its consequential damage by suppressing/regulating a series of deformations (for example, a curved shape) based on the forceful restraint of the optical fiber support even though a variety of factors (for example, weight, contact with tissue in body) are applied to the optical fiber during the fat removal surgery, thereby allowing the fat removal surgical operator (for example, a fat removal surgery medical professional) to normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

### [Description of Drawings]

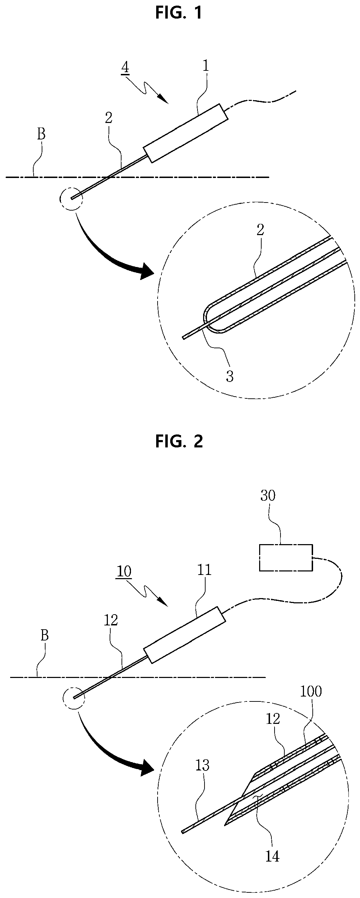

FIGS. 1 and 2 are diagrams conceptually showing a fat-removing surgical instrument according to the related art.
FIG. 3 is a diagram conceptually showing a deformed shape of an optical fiber under the regime of a fat-removing surgical instrument according to the related art.
FIG. 4 is a diagram conceptually showing a fat-removing surgical instrument according to the present disclosure.
FIGS. 5 to 28 are diagrams conceptually showing shapes of an optical fiber support according to each embodiment of the present disclosure.

### [Best Mode]

A fat-removing surgical instrument includes a wave-guide tube defining a lipolyzed fat suction passage for the suction of lipolyzed fat and configured to emit high frequency during a fat removal surgery, an optical fiber installed on the lipolyzed fat suction passage of the wave-guide tube to emit a laser during the fat removal surgery and an optical fiber support made of an insulating material and fixed to the wave-guide tube to support the optical fiber in a restrained state to prevent deformation of the optical fiber and contact with the wave-guide tube, wherein the optical fiber support includes an optical fiber hold pack extended along the wave-guide tube and covering around the optical fiber to restrain deformation of the optical fiber, and a hold pack fixing block protruded from the optical fiber hold pack and fixed to the wave-guide tube to induce the optical fiber received in the optical fiber hold pack to securely hang at the center of the lipolyzed fat suction passage.

### [Mode for Invention]

Hereinafter, a fat-removing surgical instrument according to an embodiment of the present disclosure will be described in more detail below with reference to the accompanying drawings.

As shown in FIG. 4, the fat-removing surgical instrument 200 according to an embodiment of the present disclosure assumes a systematically combined configuration of a handle 201, a wave-guide tube 212 extended and installed in the frontward direction of the handle 201, defining a lipolyzed fat suction passage 240 for the suction of lipolyzed fat and configured to emit high frequency during the fat removal surgery, and an optical fiber 230 installed on the lipolyzed fat suction passage 240 of the wave-guide tube 212 and having an end exposed in the frontward direction of the wave-guide tube 212 to emit a laser during the fat removal surgery. In this case, a high frequency cable (not shown) may be additionally embedded in the wave-guide tube 212 and electrically connected to a fat removal control terminal 230 to emit the high frequency.

The handle 201 plays a role in guiding the fat removal surgical operator (for example, a fat removal surgery medical professional) to easily handle (for example, grasp in the hand) the fat-removing surgical instrument 200, and of course, a variety of modifications may be made to the size, appearance design and incidental structure of the handle 201 according to situations.

The fat removal control terminal 230 includes a high frequency processing module, a laser processing module and a lipolyzed fat suction processing module, and is electrically/physically connected to the wave-guide tube 212, the optical fiber 230 and the lipolyzed fat suction passage 240 with the handle 201.

Under this situation, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and eventually, the fat removal surgical operator (for example, a fat removal surgery medical professional) can perform <effective fat removal surgery involving heating a localized area using the laser to rupture, breaking down the ruptured area using the high frequency and sucking up the broken fat crystals quickly>, for example, under an environment in which simultaneous emission of high frequency and laser and simultaneous suction of lipolyzed fat are performed together.

In this instance, the wave-guide tube 212 may further include an external lipolyzed fat transfer guide hole (not shown) to guide the lipolyzed fat broken down during the fat removal surgery into the lipolyzed fat suction passage 240 in the wave-guide tube 212.

Additionally, an insulation means 300, for example, an insulation tube and an insulation coating layer, may be additionally inserted/coated/placed in the wave-guide tube 212 to electrically separate the wave-guide tube 212 from the optical fiber 230 and prevent the high frequency from the wave-guide tube 212 from traveling to the optical fiber 230.

Additionally, an insulation tool (not shown) may be additionally mounted at the boundary of the body B through which the wave-guide tube 212 passes to prevent electrical/physical contact between the wave-guide tube 212 and the boundary of the body B to prevent damage to the body B from the heat generated from the wave-guide tube 212 and the high frequency emitted from the wave-guide tube 212 (hereinafter, it is equally applied to all the following embodiments).

If no action is taken during the fat removal surgery of the present disclosure, the optical fiber 230 extended along the wave-guide tube 212 unavoidably suffers a series of deformations (for example, a curved shape) due to a variety of factors (for example, weight, contact with tissue in human body), and in turn, inevitably comes into direct contact with the wave-guide tube 212 (or the insulation means in the wave-guide tube) at different points, and as a result, strong high frequency emitted from the wave-guide tube 230 is directly applied to the corresponding optical fiber 230, causing a serious problem that the optical fiber 230 breaks down into pieces and is destroyed.

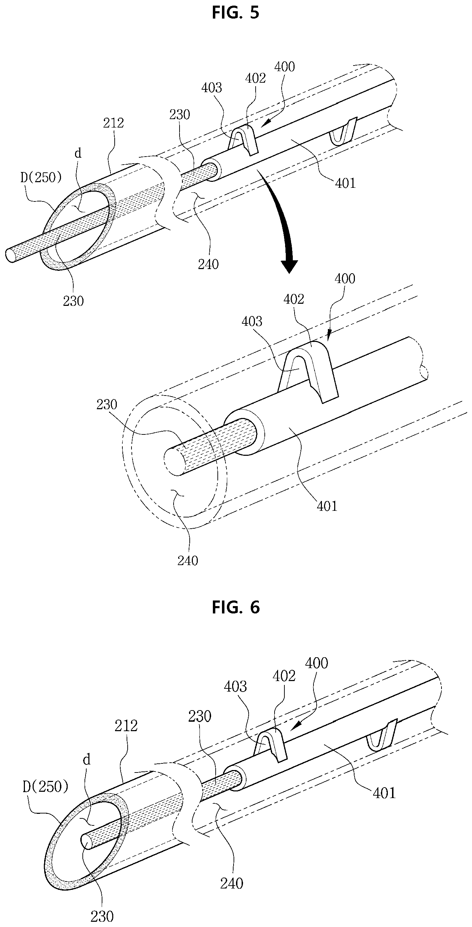

Under this critical situation, as shown in FIGS. 5 to 27, in each embodiment of the present disclosure, an action is taken to additionally place/provide, on the lipolyzed fat suction passage 240 of the wave-guide tube 212, optical fiber supports 400, 410, 420, 430, 440, 450, 460, 470, 480 made of an insulating material and fixed to the wave-guide tube 212 to support the optical fiber 230 in a restrained state to prevent deformation of the optical fiber and contact with the wave-guide tube.

Of course, under a situation in which the optical fiber supports 400, 410, 420, 430, 440, 450, 460, 470, 480 are additionally disposed, it is possible to effectively avoid the direct contact with the wave-guide tube 212 and its consequential damage by suppressing/regulating a series of deformations (for example, a curved shape) based on the forceful restraint of the optical fiber supports 400, 410, 420, 430, 440, 450, 460, 470, 480 even though a variety of factors (for example, weight, contact with tissue in body) are applied to the optical fiber 230 during the fat removal surgery.

In this instance, the optical fiber supports 400, 410, 420, 430, 440, 450, 460, 470, 480 are made of an insulating material having a good current flow prevention effect, such as plastics, rubber, leather, glass and ceramics.

Here, it is a very important technical factor in the embodiment of the present disclosure that the optical fiber supports 400, 410, 420, 430, 440, 450, 460, 470, 480 are made of an insulating material such as plastics, rubber, leather, glass and ceramics.

It is because in case that the optical fiber supports 400, 410, 420, 430, 440, 450, 460, 470, 480 of the present disclosure are made of any other material, for example, a metallic material, not the insulating material (for example, plastics, rubber, leather, glass and ceramics), the optical fiber supports 400, 410, 420, 430, 440, 450, 460, 470, 480 cannot block the high frequency emitted from the wave-guide tube 212, so the corresponding high frequency (i.e., strong electromagnetic waves) is transmitted to the optical fiber 230, and eventually, the optical fiber 230 cannot avoid serious damage caused by the high frequency.

Hereinafter, the detailed configuration and operation of the optical fiber supports 400, 410, 420, 430, 440, 450, 460, 470, 480 according to each embodiment of the present disclosure will be described in detail.

To begin with, as shown in FIG. 5, the optical fiber support 400 according to an embodiment of the present disclosure assumes a systematically combined configuration of an optical fiber hold pack 401 extended along the wave-guide tube 212 and covering around the optical fiber 230 to restrain deformation of the optical fiber 230, and a hold pack fixing block 402 protruded from the optical fiber hold pack 401 and fixed to the wave-guide tube 212 to induce the optical fiber 230 received in the optical fiber hold pack 401 to securely hang at or near the center of the lipolyzed fat suction passage 240.

In this case, the optical fiber hold pack 401 is fixed to the hold pack fixing block 402 and disposed at or near the center of the lipolyzed fat suction passage 240, and under this situation, the optical fiber hold pack 401 and the hold pack fixing block 402 are made of plastics, rubber, leather, glass and ceramics having a good current flow prevention effect as is well known.

Here, the hold pack fixing block 402 is fixed to the wave-guide tube 212, for example, through a variety of coupling means such as an insulating adhesive, an insulating adhesive tape and a nut/bolt coupler of an insulating material (of course, a variety of modifications may be made to the method of fixing the hold pack fixing block 402 to the wave-guide tube 212 and the coupling means according to situations).

In this instance, a variety of modifications may be made to the shape of the optical fiber hold pack 401, for example, circular, angular or any other shapes according to situations, and a variety of modifications may be also made to 'the range of coverage of the optical fiber hold pack 401 on the optical fiber 230' according to situations.

Additionally, the optical fiber hold pack 401 may have a similar diameter to the optical fiber 230 to cover the corresponding optical fiber 230 somewhat tightly, and according to situations, may have a diameter that is a little larger than the optical fiber 230 (for example, 110% of the diameter of the optical fiber 230) to cover the corresponding optical fiber 230 somewhat loosely.

Here, a variety of modifications may be also made to the installation location of the hold pack fixing block 402, for example, near two ends of the wave-guide tube 212, at the center of the wave-guide tube 212, at 1/3 location from one end of the wave-guide tube 212 or any other location according to situations, and a variety of modifications may be also made to the number of the hold pack fixing blocks 402 installed, for example, 2, 3, 4 and so on according to situations, and further, a variety of modification may be also made to the shape of the hold pack fixing block 402 according to situations.

In this instance, according to situations, the hold pack fixing block 402 and the optical fiber hold pack 401 may be manufactured into an integrally molded product, and after the hold pack fixing block 402 is manufactured as a separate molded product, the hold pack fixing block 402 may be integrally coupled with the optical fiber hold pack 401, for example, through a series of coupling means such as an insulating adhesive, an insulating adhesive tape and a nut/bolt fastener of an insulating material.

Additionally, a variety of modifications may be made to the protrusion direction of the hold pack fixing block 402, for example, the upward direction of the optical fiber 230, the downward direction of the optical fiber 230, the leftward direction of the optical fiber 230, the rightward direction of the optical fiber 230, 45° direction of the optical fiber 230 or the like according to situations.

In particular, since the hold pack fixing block 402 occupies only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the hold pack fixing block 402. In this case, a lipolyzed fat passing guide hole 403 may be additionally disposed in the hold pack fixing block 402 to support smoother passing of lipolyzed fat.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold pack 401 fixes the optical fiber 230 hanging at or near the center of the lipolyzed fat suction passage 240 and tightly covers around the corresponding optical fiber 230, thereby implementing the restraint mechanism for hindering deformation of the optical fiber 230.

Of course, under the restraint mechanism of the optical fiber hold pack 401, the optical fiber 230 may be located at or near the center of the lipolyzed fat suction passage 240 that may be least affected by the high frequency from the wave-guide tube 212 to easily avoid unnecessary deformation (for example, formation a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under the above-described embodiment environment of the present disclosure, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, even under the regime of the present disclosure, since the optical fiber 230 goes over the transverse area d of the wave-guide tube 212 and its end is exposed in the frontward direction of the wave-guide tube 12, when a large volume of strong high frequency is outputted in the frontward direction of the wave-guide tube 212 from the tip D of the wave-guide tube 212 inside the human body, strong electromagnetic shocks of the corresponding high frequency cause a serious problem that the optical fiber 230 breaks down into pieces and is destroyed.

Under this critical situation, the present disclosure takes an action to additionally coat an insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

In addition to the above-described action, the present disclosure may take an action to place the end of the optical fiber 230 inside of the transverse area d of the wave-guide tube 212 inside the human body as shown in FIG. 6.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

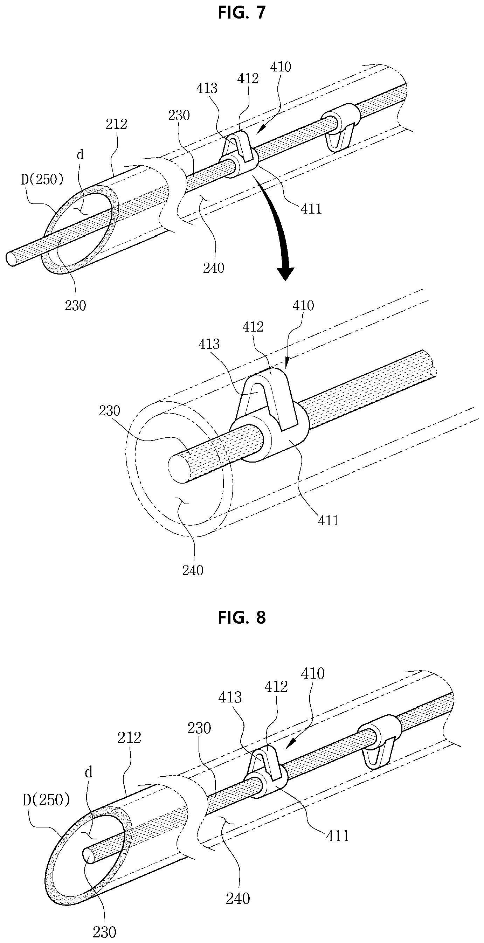

Meanwhile, as shown in FIG. 7, the optical fiber support 410 according to another embodiment of the present disclosure assumes a systematically combined configuration of an optical fiber hold piece 411 covering around the optical fiber 230 while occupying part of the optical fiber 230 to restrain deformation of the optical fiber 230 and a hold piece fixing block 412 protruded from the optical fiber hold piece 411 and fixed to the wave-guide tube 212 to induce the optical fiber 230 received in the optical fiber hold piece 411 to securely hang at the center of the lipolyzed fat suction passage 240.

In this case, the optical fiber hold piece 411 is fixed by the hold piece fixing block 412 and located at or near the center of the lipolyzed fat suction passage 240, and under this situation, the optical fiber hold piece 411 and the hold piece fixing block 412 are made of plastics, rubber, leather, glass and ceramics having a good current flow prevention effect as is well known.

Here, the hold piece fixing block 412 is fixed to the wave-guide tube 212, for example, through a variety of coupling means such as an insulating adhesive, an insulating adhesive tape and a nut/bolt fastener of an insulating material (of course, a variety of modifications may be made to the method of fixing the hold piece fixing block 412 to the wave-guide tube 212 and the coupling means according to situations).

In this instance, a variety of modifications may be made to the shape of the optical fiber hold piece 411, for example, circular, angular or any other shapes according to situations, and a variety of modifications may be also made to 'the range of coverage of the optical fiber hold piece 411 on the optical fiber 230' according to situations.

Additionally, the optical fiber hold piece 411 may have a similar diameter to the optical fiber 230 to cover the corresponding optical fiber 230 somewhat tightly, and according to situations, may have a diameter that is a little larger than the optical fiber 230 (for example, 110% of the diameter of the optical fiber 230) to cover the corresponding optical fiber 230 somewhat loosely.

Here, a variety of modification may be also made to the installation location of the optical fiber hold piece 411, for example, near two ends of the wave-guide tube 212, at the center of the wave-guide tube 212, at 1/3 location from one end of the wave-guide tube 212 or any other location according to situations, and a variety of modification may be also made to the number of the optical fiber hold pieces 411 installed, for example, 2, 3, 4 and so on according to situations, and further, a variety of modification may be also made to the shape of the hold piece fixing block 412 according to situations.

In this instance, according to situations, the hold piece fixing block 412 and the optical fiber hold piece 411 may be manufactured into an integrally molded product, and after the hold piece fixing block 412 is manufactured as a separate molded product, the hold piece fixing block 412 may be integrally coupled with the optical fiber hold piece 411, for example, through a series of coupling means such as an insulating adhesive, an insulating adhesive tape and a nut/bolt fastener of an insulating material.

Additionally, a variety of modifications may be made to the protrusion direction of the hold piece fixing block 412, for example, the upward direction of the optical fiber 230, the downward direction of the optical fiber 230, the leftward direction of the optical fiber 230, the rightward direction of the optical fiber 230, 45° direction of the optical fiber 230 or the like according to situations.

In particular, since the hold piece fixing block 412 occupies only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the hold piece fixing block 412. In this case, a lipolyzed fat passing guide hole 413 may be additionally disposed in the hold piece fixing block 412 to support smoother passing of lipolyzed fat.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold piece 411 fixes the optical fiber 230 hanging at or near the center of the lipolyzed fat suction passage 240 and tightly covers around the corresponding optical fiber 230 in part, thereby implementing the restraint mechanism for hindering deformation of the optical fiber 230.

Of course, under this restraint mechanism of the optical fiber hold piece 411, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

In addition to the above-described action, another embodiment of the present disclosure may take an action to place the end of the optical fiber 230 inside of the transverse area d of the wave-guide tube 212 inside the human body as shown in FIG. 8.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

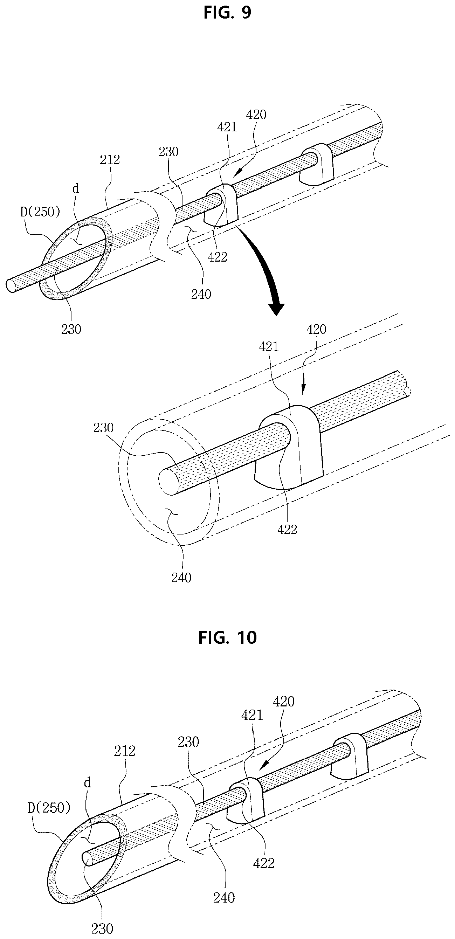

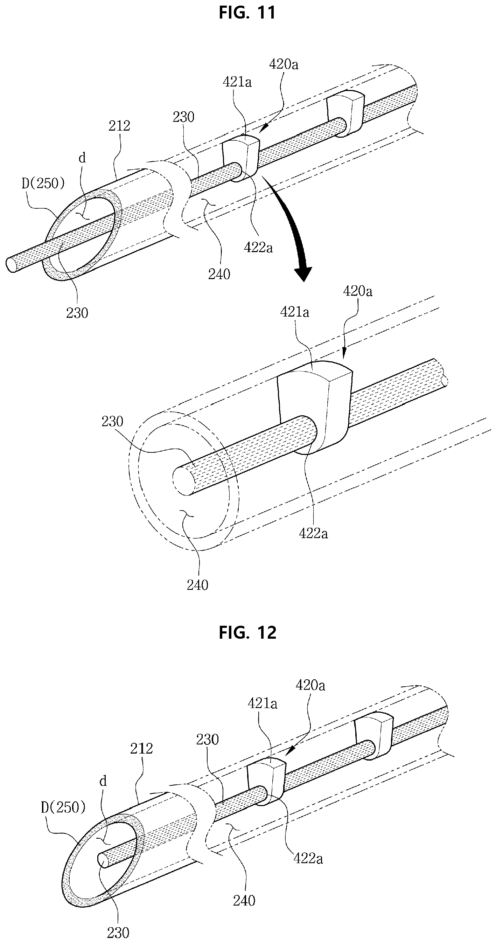

Meanwhile, as shown in FIGS. 9 and 11, the optical fiber supports 420, 420a according to another embodiment of the present disclosure assume a systematically combined configuration of body blocks 421, 421a fixed to the wave-guide tube 212 and protruded from the wave-guide tube 212 toward the lipolyzed fat suction passage 240, and optical fiber passing guide holes 422, 422a formed by making parts of the body blocks 421, 421a open, through which the optical fiber 230 passes through the body blocks 421, 421a, to induce the optical fiber 230 to securely hang at the center of the lipolyzed fat suction passage 240 with its deformation being restrained by the body blocks 421, 421a.

In this case, the optical fiber passing guide holes 422, 422a which make the body blocks 421, 421a open are located at or near the center of the lipolyzed fat suction passage 240, and under this situation, the body blocks 421, 421a are made of plastics, rubber, leather, glass and ceramics having a good current flow prevention effect.

In this instance, the body blocks 421, 421a may assume a structure in which they are installed upright in the upward direction from bottom of the wave-guide tube 212 (the case shown in FIG. 9), and may assume a structure in which they are installed hanging in the downward direction from top of the wave-guide tube 212 (the case shown in FIG. 11).

Here, the body blocks 421, 421a are fixed to the wave-guide tube 212, for example, through a variety of coupling means such as an insulating adhesive, an insulating adhesive tape and a nut/bolt fastener of an insulating material (of course, a variety of modifications may be made to the method of fixing the body blocks 421, 421a to the wave-guide tube 212 and the coupling means according to situations).

In this instance, a variety of modifications may be made to the shape of the body blocks 421, 421a according to situations, and a variety of modifications may be also made to 'the range of coverage of the body blocks 421, 421a on the optical fiber 230' according to situations.

Additionally, the optical fiber passing guide holes 422, 422a may have a similar diameter to the optical fiber 230 to cover the corresponding optical fiber 230 somewhat tightly, and according to situations, may have a diameter that is a little larger than the optical fiber 230 (for example, 110% of the diameter of the optical fiber 230) to cover the corresponding optical fiber 230 somewhat loosely.

Here, a variety of modifications may be also made to the installation location of the body blocks 421, 421a, for example, near two ends of the wave-guide tube 212, at the center of the wave-guide tube 212, at 1/3 location from one end of the wave-guide tube 212 or any other location according to situations, and a variety of modifications may be also made to the number of the body blocks 421, 421a installed, for example, 2, 3, 4 and so on according to situations.

In this instance, since the body blocks 421, 421a occupy only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the body blocks 421, 421a. In this case, a lipolyzed fat passing guide hole (not shown) may be additionally disposed in the body blocks 421, 421a to support smoother passing of lipolyzed fat.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the body blocks 421, 421a fix the optical fiber 230 hanging at or near the center of the lipolyzed fat suction passage 240, and tightly cover around the corresponding optical fiber 230 in part, thereby implementing the restraint mechanism for hindering deformation of the optical fiber 230.

Of course, under the restraint mechanism of the body blocks 421, 421a, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

In addition to the above-described action, in another embodiment of the present disclosure, an action may be taken to place the end of the optical fiber 230 inside of the transverse area d of the wave-guide tube 212 inside the human body as shown in FIGS. 10 and 12.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

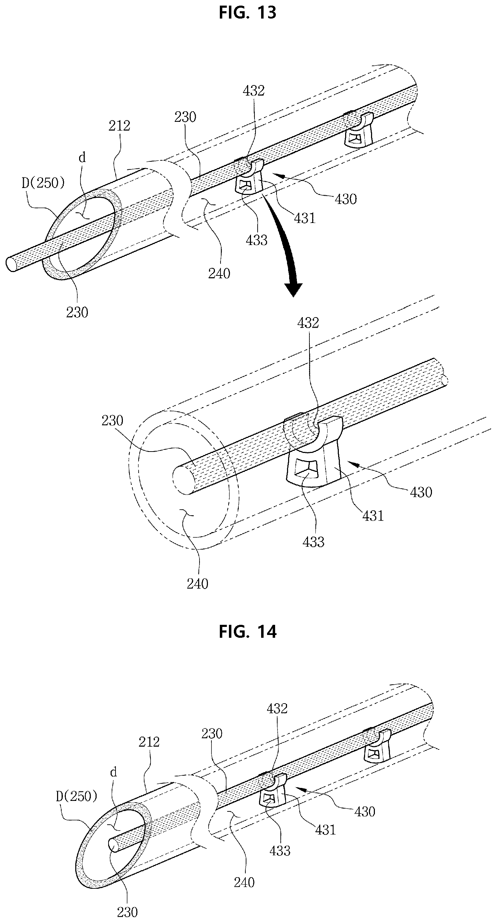

Meanwhile, as shown in FIG. 13, the optical fiber support 430 according to another embodiment of the present disclosure assumes a systematically combined configuration of an optical fiber hold ring 432 having a shape of a 'U'-shaped ring, part of which is open, and covering around the optical fiber 230 to restrain deformation of the optical fiber 230, and a base block 431 integrally connected with the optical fiber hold ring 432 and fixed to the wave-guide tube 212 to induce the optical fiber 230 passing through the optical fiber hold ring 432 to securely hang (or suspended) at the center of the lipolyzed fat suction passage 240.

In this case, the optical fiber hold ring 432 is supported/fixed by the base block 431 and located at or near the center of the lipolyzed fat suction passage 240, and under this situation, the optical fiber hold ring 432 and the base block 431 are made of plastics, rubber, leather, glass and ceramics having a good current flow prevention effect as is well known.

Here, the base block 431 is fixed to the wave-guide tube 212, for example, through a variety of coupling means such as an insulating adhesive, an insulating adhesive tape and a nut/bolt fastener of an insulating material (of course, a variety of modifications may be made to the method of fixing the base block 431 to the wave-guide tube 212 and the coupling means according to situations).

In this instance, a variety of modifications may be made to the shape of the optical fiber hold ring 432 and the base block 431 according to situations.

Additionally, the optical fiber hold ring 432 and the base block 431 may be disposed upright in the upward direction from bottom of the wave-guide tube 212, and according to situations, may be disposed hanging in the downward direction from top of the wave-guide tube 212.

Here, a variety of modifications may be also made to the installation location of the optical fiber hold ring 432 and the base block 431, for example, near two ends of the wave-guide tube 212, at the center of the wave-guide tube 212, at 1/3 location from one end of the wave-guide tube 212 or any other location according to situations, and a variety of modifications may be also made to the number of the optical fiber hold rings 432 and the base blocks 431 installed, for example, 2, 3, 4 and so on according to situations.

In this instance, according to situations, the optical fiber hold ring 432 and the base block 431 may be manufactured into an integrally molded product, and after they are manufactured as separate molded products, they may be integrally coupled to each other, for example, through a series of coupling means such as an insulating adhesive, an insulating adhesive tape and a nut/bolt fastener of an insulating material.

In particular, since the optical fiber hold ring 432 and the base block 431 occupy only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the optical fiber hold ring 432 and the base block 431. In this case, a lipolyzed fat passing guide hole 433 may be additionally disposed in the base block 431 to support smoother passing of lipolyzed fat.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold ring 432 fixes the optical fiber 230 hanging (or suspended) at or near the center of the lipolyzed fat suction passage 240 and tightly covers around the corresponding optical fiber 230 in part, thereby implementing the restraint mechanism for hindering deformation of the optical fiber 230.

Of course, under the restraint mechanism of the optical fiber hold ring 432, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

In addition to the above-described action, in another embodiment of the present disclosure, an action may be taken to place the end of the optical fiber 230 inside of the transverse area d of the wave-guide tube 212 inside the human body as shown in FIG. 14.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

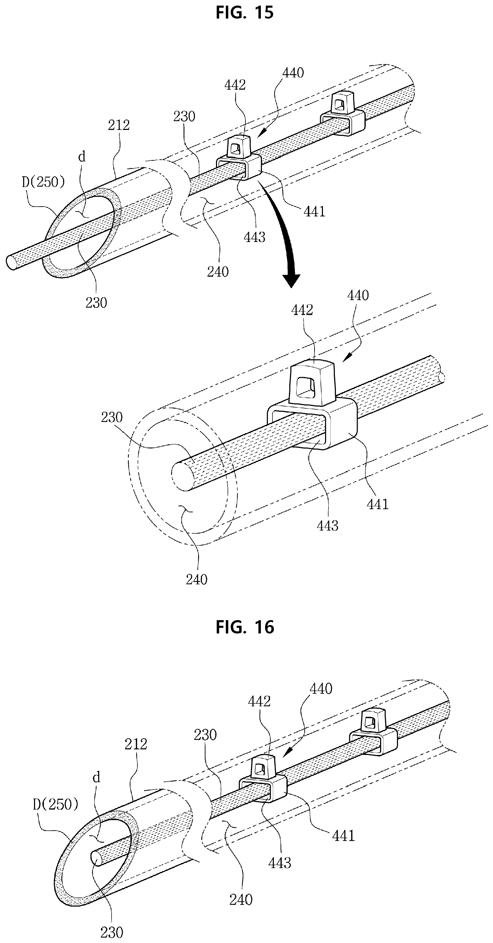

Meanwhile, as shown in FIG. 15, the optical fiber support 440 according to another embodiment of the present disclosure assumes a systematically combined configuration of an optical fiber hold box 441 covering around the optical fiber 230 through an accommodation space 443 while occupying part of the optical fiber 230 to restrain deformation of the optical fiber 230, and a hold box fixing block 442 extended from the optical fiber hold box 441 and fixed to the wave-guide tube 212 to induce the optical fiber 230 received in the optical fiber hold box 441 to securely hang at the center of the lipolyzed fat suction passage 240.

In this case, the optical fiber hold box 441 is fixed by the hold box fixing block 442 and located at or near the center of the lipolyzed fat suction passage 240, and under this situation, the optical fiber hold box 441 and the hold box fixing block 442 are made of plastics, rubber, leather, glass and ceramics having a good current flow prevention effect as is well known.

Here, the hold box fixing block 442 is fixed to the wave-guide tube 212, for example, through a variety of coupling means such as an insulating adhesive, an insulating adhesive tape and a nut/bolt fastener of an insulating material (of course, a variety of modifications may be made to the method of fixing the hold box fixing block 442 to the wave-guide tube 212 and the coupling means according to situations).

In this instance, a variety of modifications may be made to the shape of the optical fiber hold box 441 and the hold box fixing block 442, for example, circular, angular or any other shapes according to situations, and a variety of modifications may be also made to 'the range of coverage of the optical fiber hold box 441 on the optical fiber 230' according to situations.

Here, a variety of modifications may be also made to the installation location of the optical fiber hold box 441 and the hold box fixing block 442, for example, near two ends of the wave-guide tube 212, at the center of the wave-guide tube 212, at 1/3 location from one end of the wave-guide tube 212 or any other location according to situations, and a variety of modifications may be also made to the number of the optical fiber hold boxes 441 and the hold box fixing blocks 442 installed, for example, 2, 3, 4 and so on according to situations.

In this instance, according to situations, the optical fiber hold box 441 and the hold box fixing block 442 may be manufactured into an integrally molded product, and after they are manufactured as separate molded products, they may be integrally coupled to each other, for example, through a series of coupling means such as an insulating adhesive, an insulating adhesive tape and a nut/bolt fastener of an insulating material.

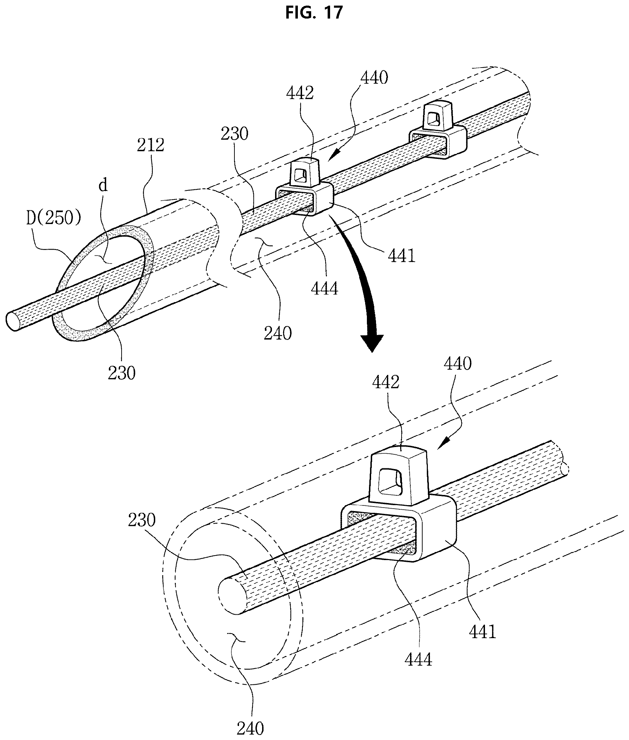

Additionally, as shown in FIG. 17, an insulating filler 444 may be additionally disposed in the optical fiber hold box 441 to prevent the movement of the received optical fiber 230 and gaps.

In particular, since the optical fiber hold box 441 and the hold box fixing block 442 occupy only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the optical fiber hold box 441 and the hold box fixing block 442. Also in this case, a lipolyzed fat passing guide hole may be additionally disposed in the hold box fixing block 442 to support smoother passing of lipolyzed fat.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold box 441 fixes the optical fiber 230 hanging at or near the center of the lipolyzed fat suction passage 240 and partially covers around the corresponding optical fiber 230, thereby implementing the restraint mechanism for hindering deformation of the optical fiber 230.

Of course, under the restraint mechanism of the optical fiber hold box 441, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment environment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

In addition to the above-described action, in another embodiment of the present disclosure, as shown in FIG. 16, an action may be taken to place the end of the optical fiber 230 inside of the transverse area d of the wave-guide tube 212 inside the human body.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

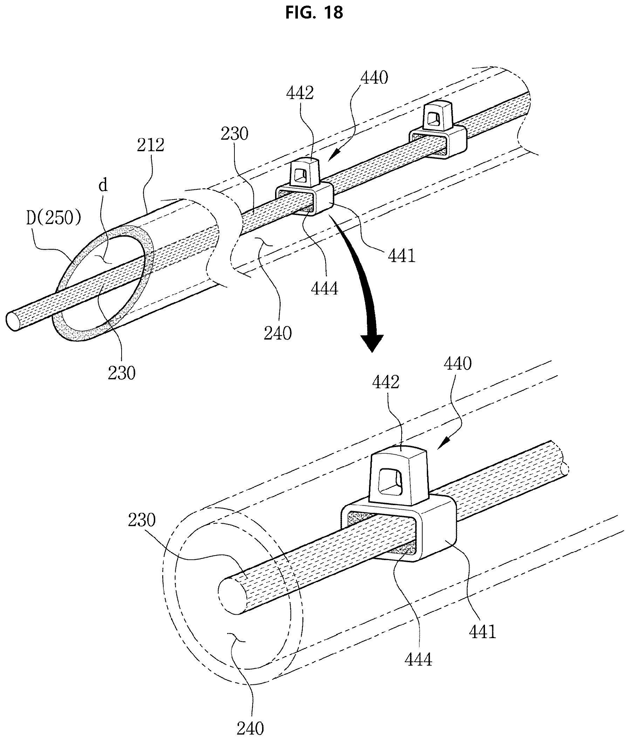

Meanwhile, as shown in FIG. 18, the optical fiber support 450 according to another embodiment of the present disclosure assumes a systematically combined configuration of an optical fiber hold pack 451 extended along the wave-guide tube 212 and covering around the optical fiber 230 to restrain deformation of the optical fiber 230 and a hold pack fixing string 452 connected to the optical fiber hold pack 451 and fixed to the wave-guide tube 212 to induce the optical fiber 230 received in the optical fiber hold pack 401 to securely hang at or near the center of the lipolyzed fat suction passage 240.

In this case, the optical fiber hold pack 451 is fixed by the hold pack fixing string 452 and located at or near the center of the lipolyzed fat suction passage 240, and under this situation, the optical fiber hold pack 451 and the hold pack fixing string 452 are made of plastics, rubber, leather, glass and ceramics having a good current flow prevention effect as is well known.

Here, the hold pack fixing string 452 is fixed to the wave-guide tube 212 and the optical fiber hold pack 451, for example, through a variety of coupling means such as an insulating adhesive and an insulating adhesive tape (of course, a variety of modifications may be made to the method of fixing the hold pack fixing string 452 to the wave-guide tube 212 and the optical fiber hold pack 451 and the coupling means according to situations).

In this instance, a variety of modifications may be made to the shape of the optical fiber hold pack 451, for example, circular, angular or any other shapes according to situations, and a variety of modifications may be also made to 'the range of coverage of the optical fiber hold pack 451 on the optical fiber 230' according to situations.

Additionally, the optical fiber hold pack 451 may have a similar diameter to the optical fiber 230 to cover the corresponding optical fiber 230 somewhat tightly, and according to situations, may have a diameter that is a little larger than the optical fiber 230 (for example, 110% of the diameter of the optical fiber 230) to cover the corresponding optical fiber 230 somewhat loosely.

Here, a variety of modifications may be also made to the installation location of the hold pack fixing string 452, for example, near two ends of the wave-guide tube 212, at the center of the wave-guide tube 212, at 1/3 location from one end of the wave-guide tube 212 or any other location according to situations, and a variety of modifications may be also made to the number of the hold pack fixing strings 452 installed, for example, 2, 3, 4 and so on according to situations.

Of course, since the hold pack fixing string 452 occupies only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold pack 451 fixes the optical fiber 230 hanging at or near the center of the lipolyzed fat suction passage 240 and tightly covers around the corresponding optical fiber 230, thereby implementing the restraint mechanism for hindering deformation of the optical fiber 230.

Of course, under the restraint mechanism of the optical fiber hold pack 451, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety factors (for example, weight, contact of the optical fiber with tissue in human body) are applied to the optical fiber 230 during the fat removal surgery.

Obviously, under the above-described embodiment environment of the present disclosure, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

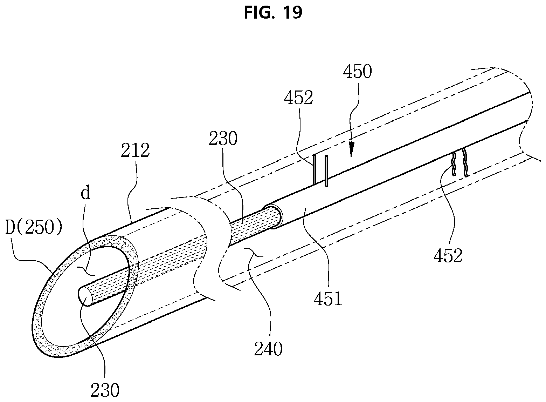

In addition to the above-described action, in another embodiment of the present disclosure, as shown in FIG. 19, an action may be taken to place the end of the optical fiber 230 inside of the transverse area d of the wave-guide tube 212 inside the human body.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

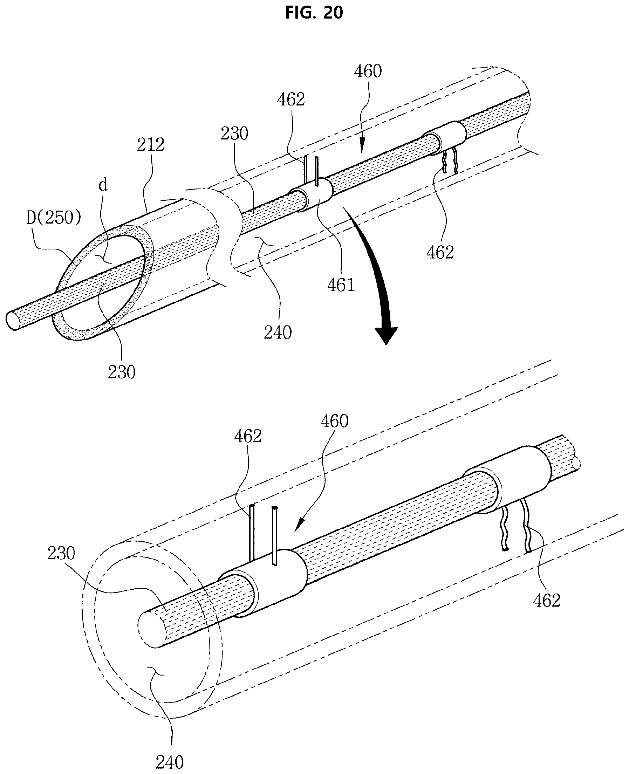

Meanwhile, as shown in FIG. 20, the optical fiber support 460 according to another embodiment of the present disclosure assumes a systematically combined configuration of an optical fiber hold piece 461 covering around the optical fiber 230 while occupying part of the optical fiber 230 to restrain deformation of the optical fiber 230 and a hold piece fixing string 462 connected to the optical fiber hold piece 461 and fixed to the wave-guide tube 212 to induce the optical fiber 230 received in the optical fiber hold piece 461 to securely hang at the center of the lipolyzed fat suction passage 240.

In this case, the optical fiber hold piece 461 is fixed by the hold piece fixing string 462 and located at or near the center of the lipolyzed fat suction passage 240, and under this situation, the optical fiber hold piece 461 and the hold piece fixing string 462 are made of plastics, rubber, leather, glass and ceramics having a good current flow prevention effect as is well known.

Here, the hold piece fixing string 462 is connected/fixed to the wave-guide tube 212 and the optical fiber hold piece 461, for example, through a variety of coupling means such as an insulating adhesive and an insulating adhesive tape (of course, a variety of modifications may be made to the method of fixing the hold piece fixing block 412 to the wave-guide tube 212 and the optical fiber hold piece 461 and the coupling means according to situations).

In this instance, a variety of modifications may be made to the shape of the optical fiber hold piece 461, for example, circular, angular or any other shapes according to situations, and a variety of modifications may be also made to 'the range of coverage of the optical fiber hold piece 461 on the optical fiber 230' according to situations.

Additionally, the optical fiber hold piece 461 may have a similar diameter to the optical fiber 230 to cover the corresponding optical fiber 230 somewhat tightly, and according to situations, may have a diameter that is a little larger than the optical fiber 230 (for example, 110% of the diameter of the optical fiber 230) to cover the corresponding optical fiber 230 somewhat loosely.

Here, a variety of modifications may be made to the installation location of the optical fiber hold piece 461 and the hold piece fixing string 462, for example, near two ends of the wave-guide tube 212, at the center of the wave-guide tube 212, at 1/3 location from one end of the wave-guide tube 212 or any other location according to situations, and a variety of modifications may be also made to the number of the optical fiber hold pieces 461 and the hold piece fixing strings 462 installed, for example, 2, 3, 4 and so on according to situations.

Of course, since the hold piece fixing string 462 occupies only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold piece 461 fixes the optical fiber 230 hanging at or near the center of the lipolyzed fat suction passage 240 and tightly covers around the corresponding optical fiber 230 in part, thereby implementing the restraint mechanism for hindering deformation of the optical fiber 230.

Of course, under the restraint mechanism of the optical fiber hold piece 461, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment environment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

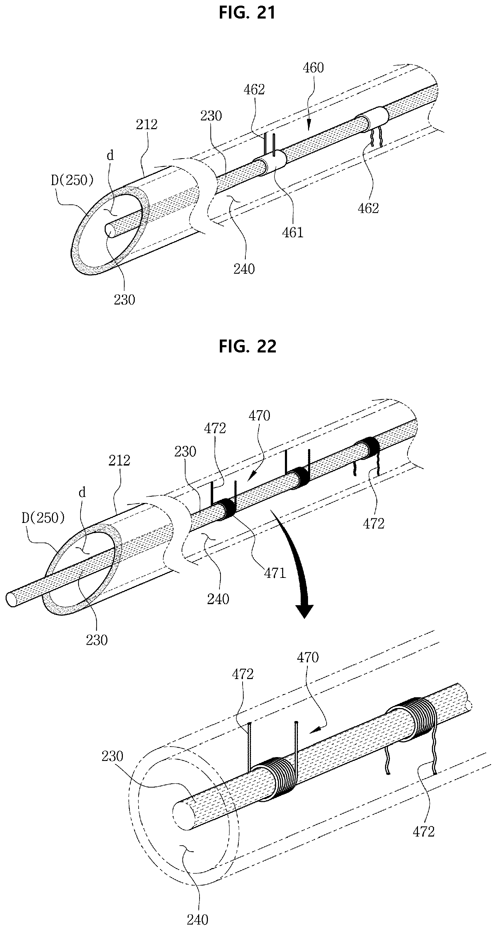

In addition to the above-described action, in another embodiment of the present disclosure, an action may be taken to place the end of the optical fiber 230 inside of the transverse area d of the wave-guide tube 212 inside the human body as shown in FIG. 21.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

Meanwhile, as shown in FIG. 22, the optical fiber support 470 according to another embodiment of the present disclosure includes a plurality of optical fiber fixing strings 472 continuously wound on the optical fiber 230 to form a hank 471 to restrain deformation of the optical fiber 230, and having two ends fixed to the wave-guide tube 212 to induce the optical fiber 230 to securely hang at (or near) the center of the lipolyzed fat suction passage 240.

In this case, the optical fiber fixing string 472 is made of rubber and leather having a good current flow prevention effect.

Here, the optical fiber fixing string 472 is connected/fixed to the wave-guide tube 212, for example, through a variety of coupling means such as an insulating adhesive and an insulating adhesive tape (of course, a variety of modifications may be made to the method of fixing the optical fiber fixing string 472 to the wave-guide tube 212 and the coupling means according to situations).

In this instance, a variety of modifications may be made to 'the range of coverage of the hank 471 of the optical fiber fixing string 472 on the optical fiber 230' according to situations.

Here, a variety of modifications may be made to the installation location of the optical fiber fixing string 472, for example, near two ends of the wave-guide tube 212, at the center of the wave-guide tube 212, at 1/3 location from one end of the wave-guide tube 212 or any other location according to situations, and a variety of modifications may be also made to the number of the optical fiber fixing strings 472 installed, for example, 2, 3, 4 and so on according to situations.

Of course, since the optical fiber fixing string 472 occupies only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the optical fiber fixing string 472.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber fixing string 472 fixes the optical fiber 230 hanging at or near the center of the lipolyzed fat suction passage 240 and tightly covers around the corresponding optical fiber 230 in part through the hank 471, thereby implementing the restraint mechanism for hindering deformation of the optical fiber 230.

Of course, under the restraint mechanism of the hank 471 of the optical fiber fixing string 472, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally operate the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

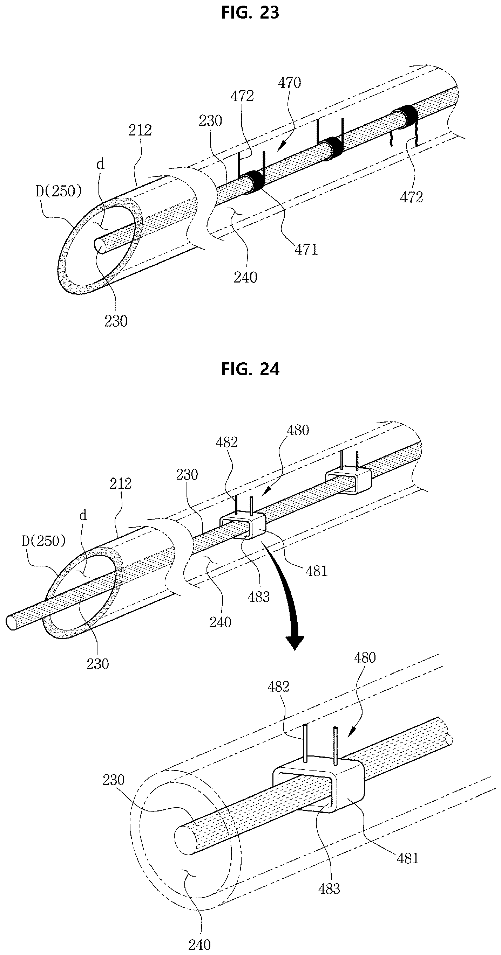

In addition to the above-described action, in another embodiment of the present disclosure, an action may be taken to place the end of the optical fiber 230 inside of the transverse area d of the wave-guide tube 212 inside the human body as shown in FIG. 23.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

Meanwhile, as shown in FIG. 24, the optical fiber support 480 according to another embodiment of the present disclosure assumes a systematically combined configuration of an optical fiber hold box 481 covering around the optical fiber 230 through an accommodation space 483 while occupying part of the optical fiber 230 to restrain deformation of the optical fiber 230, and a hold box fixing string 482 connected to the optical fiber hold box 481 and fixed to the wave-guide tube 212 to induce the optical fiber 230 received in the optical fiber hold box 481 to securely hang at the center of the lipolyzed fat suction passage 240.

In this case, the optical fiber hold box 481 is fixed by the hold box fixing string 482 and located at or near the center of the lipolyzed fat suction passage 240, and under this situation, the optical fiber hold box 481 and the hold box fixing string 482 are made of plastics, rubber, leather, glass and ceramics having a good current flow prevention effect as is well known.

Here, the hold box fixing string 482 is fixed to the wave-guide tube 212 and the optical fiber hold box 481, for example, through a variety of coupling means such as an insulating adhesive and an insulating adhesive tape (of course, a variety of modifications may be made to the method of fixing the hold box fixing block 442 to the wave-guide tube 212 and the optical fiber hold box 481 and the coupling means according to situations).

In this instance, a variety of modifications may be made to the shape of the optical fiber hold box 481, for example, circular, angular or any other shapes according to situations, and a variety of modifications may be also made to 'the range of coverage of the optical fiber hold box 481 on the optical fiber 230' according to situations.

Here, a variety of modifications may be also made to the installation location of the optical fiber hold box 481 and the hold box fixing string 482, for example, near two ends of the wave-guide tube 212, at the center of the wave-guide tube 212, at 1/3 location from one end of the wave-guide tube 212 or any other location according to situations, and a variety of modifications may be also made to the number of the optical fiber hold boxes 481 and the hold box fixing strings 482 installed, for example, 2, 3, 4 and so on according to situations.

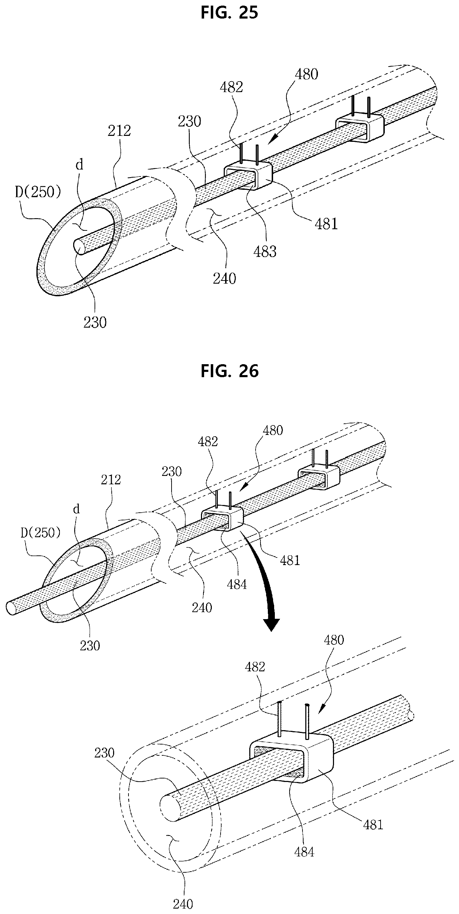

Additionally, as shown in FIG. 26, an insulating filler 484 may be additionally disposed in the optical fiber hold box 481 to prevent the movement of the received optical fiber 230 and gaps.

In particular, since the optical fiber hold box 481 occupies only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the optical fiber hold box 481.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the optical fiber hold box 481 fixes the optical fiber 230 hanging at or near the center of the lipolyzed fat suction passage 240 and partially covers around the corresponding optical fiber 230, thereby implementing the restraint mechanism for hindering deformation of the optical fiber 230.

Of course, under the restraint mechanism of the optical fiber hold box 481, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally operate the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

In addition to the above-described action, in another embodiment of the present disclosure, an action may be taken to place the end of the optical fiber 230 inside of the transverse area d of the wave-guide tube 212 inside the human body as shown in FIG. 25.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

As described above, since each embodiment of the present disclosure additionally places/provides, on the lipolyzed fat suction passage of the wave-guide tube, 'the optical fiber support made of an insulating material and fixed to the wave-guide tube to support the optical fiber in a restrained state to prevent deformation of the optical fiber and contact with the wave-guide tube', under the embodiment environment of the present disclosure, it is possible to effectively avoid the direct contact of the optical fiber with the wave-guide tube and its consequential damage by suppressing/regulating a series of deformations (for example, a curved shape) based on the forceful restraint of the optical fiber support even though a variety of factors (for example, weight, contact with tissue in human body) are applied to the optical fiber during the fat removal surgery, thereby allowing the fat removal surgical operator (for example, a fat removal surgery medical professional) to normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

The present disclosure may have a variety of modifications according to situations.

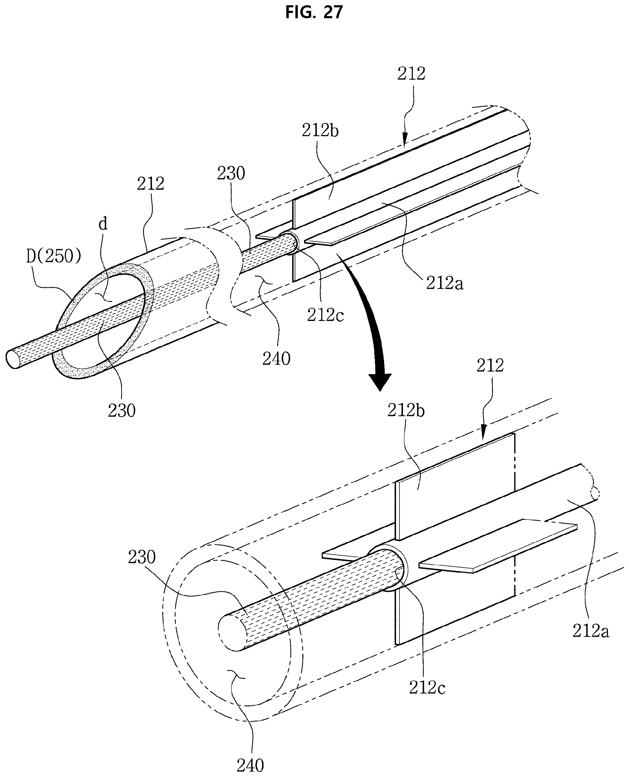

For example, as shown in FIG. 27, the present disclosure improves the internal structure of the wave-guide tube 212 to 'make the wave-guide tube 212 itself have the internal structure for suppressing deformation of the optical fiber 212 and placing the corresponding optical fiber 212 near the center of the lipolyzed fat suction passage 240'.

In this case, as shown in FIG. 27, an inner insulation tube 212a of an insulating material is installed at the center of the lipolyzed fat suction passage 240 and covers around the optical fiber 230 to restrain deformation of the optical fiber 230, and a fixing frame 212b of an insulating material is installed on the outer side of the inner insulation tube 212a and protrudes from the inner insulation tube 212a to fix the corresponding inner insulation tube 212a on the lipolyzed fat suction passage 240, and under this situation, the optical fiber 230 is inserted into an insertion hole 212c of the inner insulation tube 212a and securely hangs at (or near) the center of the lipolyzed fat suction passage 240.

In this instance, the inner insulation tube 212a and the fixing frame 212b are made of plastics, glass and ceramics having a good current flow prevention effect as is well known.

Here, since the inner insulation tube 212a and the fixing frame 212b occupy only part of the lipolyzed fat suction passage 240 at an optimal scale in the lipolyzed fat suction passage 240, it is possible to normally maintain smooth suction of lipolyzed fat passing through the corresponding lipolyzed fat suction passage 240 without unnecessary obstruction by the inner insulation tube 212a and the fixing frame 212b.

Under this situation in which the infrastructure is established, when the fat removal surgery in the subject's body B starts, the fat removal control terminal 230 operates the high frequency processing module, the laser processing module and the lipolyzed fat suction processing module, for example, to simultaneously perform the procedure of applying high frequency to the wave-guide tube 212, the procedure of applying a laser to the optical fiber 230 and the procedure of sucking up lipolyzed fat through the lipolyzed fat suction passage 240, and under this situation, the optical fiber 230 extended along the wave-guide tube 212 faces danger of a series of deformations (for example, formation of a curved shape) due to a variety of factors (for example, weight, contact with tissue in human body).

Under this dangerous situation, the inner insulation tube 212a fixes the optical fiber 230 hanging at or near the center of the lipolyzed fat suction passage 240 and covers around the corresponding optical fiber 230, thereby implementing the restraint mechanism for hindering deformation of the optical fiber 230.

Of course, under the restraint mechanism of the inner insulation tube 212a, the optical fiber 230 may be located at or near the center of the lipolyzed fat suction passage 240 that may be least affected by the high frequency from the wave-guide tube 212 to easily avoid unnecessary deformation (for example, formation of a curved shape), thereby effectively avoiding the direct contact with the wave-guide tube 212 and its consequential damage without any deformation even though a variety of factors (for example, weight, contact with tissue in human body) are applied to the optical fiber 230 during the fat removal surgery.

Obviously, under another embodiment environment of the present disclosure described above, the fat removal surgical operator (for example, a fat removal surgery medical professional) can normally perform the effective fat removal surgery in which simultaneous high frequency/laser emission and simultaneous lipolyzed fat suction can be performed together while easily avoiding serious problems caused by reckless infiltration/propagation of broken optical fiber scrap into the body.

Meanwhile, under another embodiment of the present disclosure described above, an action is taken to additionally coat the insulating material 250 (for example, a metal oxide material such as SiO₂) at the tip D of the wave-guide tube 212 inside the human body to shield the high frequency output.

Of course, under the additional coating situation of the insulating material 250 (for example, a metal oxide material such as SiO₂), it is possible to fundamentally shield the high frequency output from the tip D of the wave-guide tube 212 inside the human body, thereby easily avoiding damage caused by the influence of the high frequency under the situation in which the optical fiber 230 goes over the transverse area d of the wave-guide tube 212 and its end is exposed in the frontward direction of the wave-guide tube 12.

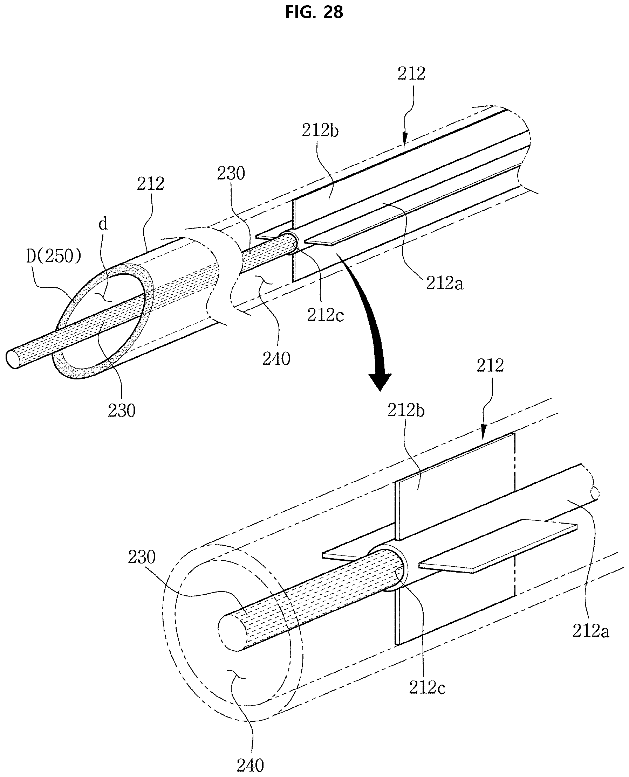

In addition to the above-described action, in another embodiment of the present disclosure, an action may be taken to place the end of the optical fiber 230 inside of the transverse area d of the wave-guide tube 212 inside the human body as shown in FIG. 28.

Of course, under this situation in which the end of the optical fiber 230 is located inside of the transverse area d of the wave-guide tube 212 inside the human body, even though a large volume of strong high frequency is outputted in the frontward direction of the wave-guide tube 212 from the tip D of the wave-guide tube 212 inside the human body, the optical fiber 230 can easily avoid the exposure, thereby easily avoiding damage caused by the influence of the high frequency.

The present disclosure is not limited to a specific field, and exerts its useful effect all over the fields requiring high frequency/laser surgery.

Additionally, although a specific embodiment of the present disclosure has been hereinabove described and illustrated, it is obvious that the present disclosure may be practiced in a variety of modified forms by those skilled in the art.

The variations should not be understood separately from the scope or technical spirit of the present disclosure and it should be understood that the variations fall within the appended claims.

### [Detailed Description of Main Elements]

200: Fat-removing surgical instrument
212: Wave-guide tube
212a: Inner insulation tube
212b: Fixing frame
230: Optical fiber
240: Lipolyzed fat suction passage
400, 410, 420, 430, 440, 450, 460, 470, 480: Optical fiber support
401, 451: Optical fiber hold pack
402: Hold pack fixing block
411, 461: Optical fiber hold piece
412: Hold piece fixing block
421: Boby block
431: Base block
432: Optical fiber hold ring
441, 481: Optical fiber hold box
442: Hold box fixing block
452: Hold pack fixing string
462: Hold piece fixing string
471: Hank
472: Optical fiber fixing string
482: Hold box fixing string

## Claims

1. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes:
an optical fiber hold pack extended along the wave-guide tube and covering around the optical fiber to restrain deformation of the optical fiber; and
a hold pack fixing block protruded from the optical fiber hold pack and fixed to the wave-guide tube to induce the optical fiber received in the optical fiber hold pack to securely hang at a center of the lipolyzed fat suction passage.

2. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes:
an optical fiber hold piece covering around the optical fiber while occupying part of the optical fiber to restrain deformation of the optical fiber; and
a hold piece fixing block protruded from the optical fiber hold piece and fixed to the wave-guide tube to induce the optical fiber received in the optical fiber hold piece to securely hang at a center of the lipolyzed fat suction passage.

3. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes:
a body block fixed to the wave-guide tube and protruded from the wave-guide tube toward the lipolyzed fat suction passage; and
an optical fiber passing guide hole formed by making part of the body block open, through which the optical fiber passes through the body block, to induce the optical fiber to securely hang at a center of the lipolyzed fat suction passage with deformation being restrained by the body block.

4. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes:
an optical fiber hold ring having a ring shape, part of which is open, and covering around the optical fiber to restrain deformation of the optical fiber; and
a base block integrally connected with the optical fiber hold ring and fixed to the wave-guide tube to induce the optical fiber passing through the optical fiber hold ring to securely hang at a center of the lipolyzed fat suction passage.

5. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes:
an optical fiber hold pack extended along the wave-guide tube and covering around the optical fiber to restrain deformation of the optical fiber; and
a hold pack fixing string connected to the optical fiber hold pack and fixed to the wave-guide tube to induce the optical fiber received in the optical fiber hold pack to securely hang at a center of the lipolyzed fat suction passage.

6. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes:
an optical fiber hold piece covering around the optical fiber while occupying part of the optical fiber to restrain deformation of the optical fiber; and
a hold piece fixing string connected to the optical fiber hold piece and fixed to the wave-guide tube to induce the optical fiber received in the optical fiber hold piece to securely hang at a center of the lipolyzed fat suction passage.

7. A fat-removing surgical instrument, comprising:
a wave-guide tube defining a lipolyzed fat suction passage for suction of lipolyzed fat and configured to emit high frequency during a fat removal surgery;
an optical fiber installed on the lipolyzed fat suction passage of the wave-guide tube and configured to emit a laser during the fat removal surgery; and
an optical fiber support made of an insulating material and fixed to the wave-guide tube to support the optical fiber in a restrained state to prevent deformation of the optical fiber and contact with the wave-guide tube,
wherein the optical fiber support includes a plurality of optical fiber fixing strings continuously wound around the optical fiber to a form a hank to restrain deformation of the optical fiber, and having two ends fixed to the wave-guide tube to induce the optical fiber to securely hang at a center of the lipolyzed fat suction passage.

8. A fat-removing surgical instrument, comprising:
a wave-guide tube defining a lipolyzed fat suction passage for suction of lipolyzed fat and configured to emit high frequency during a fat removal surgery; and
an optical fiber installed on the lipolyzed fat suction passage of the wave-guide tube and configured to emit a laser during the fat removal surgery,
wherein an inner insulation tube of an insulating material is installed at a center of the lipolyzed fat suction passage and covers around the optical fiber to restrain deformation of the optical fiber,
a fixing frame of an insulating material is installed on an outer side of the inner insulation tube and protrudes from the inner insulation tube to fix the corresponding inner insulation tube on the lipolyzed fat suction passage, and
the optical fiber is inserted into the inner insulation tube and securely hangs at the center of the lipolyzed fat suction passage.

9. The fat-removing surgical instrument according to claim 8, wherein the inner insulation tube and the fixing frame are made of plastics.

10. The fat-removing surgical instrument according to claim 1, 2, 3, 4, 5, 6, 7 or 8, wherein an insulating material is additionally coated at a tip of the wave-guide tube inside a human body to shield an output of the high frequency.

11. The fat-removing surgical instrument according to claim 1, 2, 3, 4, 5, 6, 7 or 8, wherein an end of the optical fiber is located inside of a tip region of the wave-guide tube inside a human body.
